Europäisches Patentamt

(19) European Patent Office

Office européen des brevets

(11) Veröffentlichungsnummer: **0 006 966**
**B1**

# (12) EUROPÄISCHE PATENTSCHRIFT

(45) Veröffentlichungstag der Patentschrift:
**14.10.81**

(51) Int. Cl.³: **A 61 B 5/00**

(21) Anmeldenummer: **79100787.5**

(22) Anmeldetag: **15.03.79**

(54) Vorrichtung zur Halterung im Munde von Elektroden und dergleichen Aufnehmern für biologische und physiologische Messwerte.

(30) Priorität: **23.03.78 DE 2812816**
**17.04.78 DE 2816519**

(43) Veröffentlichungstag der Anmeldung:
**23.01.80 Patentblatt 80/2**

(45) Bekanntmachung des Hinweises auf die Patenterteilung:
**14.10.81 Patentblatt 81/41**

(84) Benannte Vertragsstaaten:
**CH FR GB NL**

(56) Entgegenhaltungen:
**DE-A-2 027 530**
**DE-B-1 202 938**
**FR-A-827 720**
**FR-A-2 255 872**
**US-A-3 152 587**
**US-A-3 650 265**
**US-A-3 882 850**
**US-A-3 971 366**

(73) Patentinhaber: **Hellige GmbH,**
**Postfach 728 Heinrich-von-Stephan-Strasse 4,**
**D-7800 Freiburg (DE)**

(72) Erfinder: **Czech, Kurt, Dr. med., Schwarzenbergplatz 13,**
**A-1040 Wien (AT)**
Erfinder: **Lackner, Franz, Dr. med., Laudongasse 13,**
**A-1080 Wien (AT)**
Erfinder: **Porges, Paul, Dr. med., Oldenburggasse 26,**
**A-1230 Wien (AT)**

(74) Vertreter: **Ter Meer-Müller-Steinmeister Patentanwälte,**
**Triftstrasse 4, D-8000 München 22 (DE)**

## Vorrichtung zur Halterung im Munde von Elektroden und dergleichen Aufnehmern für biologische und physiologische Meßwerte

Die Erfindung betrifft eine Klemmvorrichtung zur Halterung von Meßwertaufnehmern, von Elektroden und dergleichen für biologische und physiologische Meßwerte im Munde eines Patienten oder allgemein eines Probanden. Mit dieser gattungsgemäß im Oberbegriff des Patentanspruchs 1 definierten Klemmvorrichtung läßt sich beispielsweise ein Meßwertaufnehmer zur transkutanen Bestimmung des Sauerstoffpartialdrucks an der Innenseite der Lippe oder an der Innenseite einer Wange auf der Mundschleimhaut befestigen.

Die Möglichkeit zur Applikation solcher Meßaufnehmer an der Mundschleimhaut ist von besonders großer praktischer Bedeutung für polarographische Meßwertaufnehmer, die zur transkutanen Bestimmung der Konzentration von Gasen, insbesondere von Sauerstoff im Blut dienen. Über diese Methode gibt die Arbeit von A. R. Huch, »Klinische und Physiologische Aspekte der Transkutanen Sauerstoffdruckmessung in der Perinatalen Medizin«, Zeitschrift für Geburtshilfe und Perinatologie, Band 179, Heft 4, 1975, Seiten 235 – 249, einen eingehenden Überblick. Die in jüngerer Zeit mit dieser Methode erzielten Fortschritte beruhen vor allem auf der gleichzeitigen Hyperämisierung der Hautschicht, an der gemessen wird, mittels einer Beheizung des nachfolgend auch als Elektrode bezeichneten Meßaufnehmers bis zu 45°C, so daß die mit ihr in Berührung stehende Haut eine Temperatur bis zu 43°C annehmen kann. Dadurch wird die Durchblutung der Hautschicht erhöht, und der Partialdruck des Sauerstoffs nimmt Werte an, die mit dem Partialdruck in den Blutgefäßen vergleichbar und deshalb gut meßbar sind. Der Sauerstoff diffundiert in bekannter Weise durch die Haut zu der polarographisch empfindlichen Elektrode des Meßwertaufnehmers, wobei der Fluß vom Druckgradienten abhängt. Auch kurzzeitige Druckschwankungen werden im allgemeinen von solchen Aufnehmern rasch und mit großer Genauigkeit erfaßt.

Während die Technik solcher polarographischer Meßwertaufnehmer hoch entwickelt ist, ergeben sich immer noch Probleme bei der möglichst ortsfesten Anlegung und Halterung der Elektrode an der Applikationsstelle. Eine ausreichend fixierte Befestigung des Meßwertaufnehmers ist insbesondere bei der Überwachung von Patienten während der Narkose erforderlich, da während der Narkose und Operation der transkutan meßbare Sauerstoffpartialdruck deutlich vom arteriellen Wert abweichen kann, insbesondere wenn der Meßwertaufnehmer, wie bisher überwiegend üblich, im Thoraxgebiet oder am Arm auf die Haut geklebt wird. Diese Abweichungen sind vor allem auf die verringerte Durchblutung der Haut in Folge von Abkühlung und Zentralisation (Drosselung der Blutversorgung unwichtiger Organe) zurückzuführen. Außerdem ist die Anbringung des Meßwertaufnehmers am Thorax in vielen Fällen aus Sterilitätsgründen unerwünscht und an den Armen wegen der meist erforderlichen Einführung von Kathetern und der Anbringung einer Manschette zur laufenden Blutdruckmessung während der Operation unbequem und störend.

Daraus ergab sich die Forderung, die Verläßlichkeit der transkutanen polarographischen Bestimmung des Sauerstoffpartialdrucks im Blut zu verbessern durch eine Applikation an Stellen des Körpers, an denen die genannten Schwierigkeiten und Mängel nicht auftreten. Insbesondere die Anbringung des Meßwertaufnehmers im Bereich des Kopfes des Patienten bietet Vorteile, weil der Kopf während der meisten Operationen zugänglich und frei bleibt, und die Entfernung vom Kopf zu den meist kopfseitig angeordneten elektronischen Überwachungsgeräten kurzgehalten werden kann. Die Mundhöhle bzw. die Mundschleimhaut wurde als ein für die Anbringung eines solchen Aufnehmers besonders gut geeigneter Bereich ermittelt. In diesem Bereich jedoch ist eine ortsfeste Fixierung des Meßwertaufnehmers mit ausreichendem, jedoch nicht zu starkem Andruck besonders schwierig.

Zur Feststellung der Sauerstoffsättigung im Blut auf unblutige Weise durch das Verfahren der Oxymetrie sind schon Klemmervorrichtungen für die Befestigung eines photoelektrischen Meßwertaufnehmers bekannt (vgl. DE-A-20 27 530 und US-A-31 52 587). Die Gestaltung dieser bekannten Klemmervorrichtungen ist jedoch sehr stark auf den vorgesehenen Applikationsort angepaßt, weil bei der Oxymetrie bestimmte periphere Gewebeteile durchleuchtet werden müssen, wofür sich vornehmlich die Ohrläppchen empfehlen. Zur Halterung eines kleinen Mikrophons in der Mundhöhle, mit welchem Atemgeräusche und dergleichen bei der Beatmung erfaßt werden können, ist aus der FR-A-22 55 872 auch bereits eine Klemmervorrichtung bekannt (vgl. insbesondere Fig. 26), die aus zwei, etwa in der Mitte gelenkig miteinander verbundenen, Klemmerarmen besteht, von denen der eine Klemmerarm wippenartig abgewinkelt ist, so daß eine ausreichende Klemmeröffnung zur leichten Befestigung des Mikrophons, beispielsweise an der Zunge oder an der Unterlippe ermöglicht ist. Andere Befestigungsvorrichtungen für Meßelektroden und dergleichen in der Mundhöhle sehen die Abstützung am in die Mundhöhle ragenden Teil des Ober- oder Unterkiefers vor (vgl. US-A-36 50 265).

Für die transkutane Bestimmung von Blutgasen mit Hilfe von polarographischen Meßwertaufnehmern ist jedoch einerseits keine Durchstrahlung erforderlich, deshalb müssen grundsätzlich auch keine Teile der Meßeinrichtung beidseitig eines Körperteils angeordnet werden. Anders als bei den bekannten Klemm- und Haltevorrichtungen für ein Mikrophon oder eine Meßelektrode in der Mundhöhle jedoch kommt es bei der polarographischen transkutanen Sauerstoffpartialdruckmessung, wie oben bereits angedeutet, andererseits entscheidend darauf an, daß der Meßwertaufnehmer mit möglichst gleichbleibendem Andruck ortsfest an der Applikationsstelle in der Mundhöhle fixiert bleibt. Mit den bisher bekannten Haltevorrichtungen ist dieser Forderung nicht zu genügen, weshalb auch bei Operationen bisher mit auf eine bestimmte Hautstelle aufgeklebten transkutanen Meßwertaufnehmern gearbeitet wurde. Die zuverlässige Fixierung eines Meßwertaufnehmers in der Mundhöhle mit ausreichendem gleichbleibendem Anpreßdruck war bisher nicht möglich.

Der Erfindung liegt damit die Aufgabe zugrunde, eine Haltevorrichtung zur Halterung von biologisch-physiologischen Meßwertaufnehmern im Munde zu schaffen, die eine ortsfeste Fixierung eines solchen Meßwertaufnehmers, beispielsweise an der Innenseite einer Wange mit genau einstellbarem und gleichbleibend gutem Anpreßdruck an der Applikationsstelle ermöglicht.

Die Erfindung besteht bei einer Klemmvorrichtung nach der im Oberbegriff des Patentanspruchs 1 erfindungsgemäß darin, daß in das außerhalb des Mundes liegende Bedienungsende eines der Klammerarme der Klemmvorrichtung eine drehbar und damit axial verstellbare Druckschraube eingesetzt ist, die gegen das gegenüberliegende Ende des anderen Klemmarms oder gegen eine beide Klemmarme haltende Halteplatte so anstellbar ist, daß ein geeigneter Klemmdruck des Meßaufnehmers gegen die Applikationsstelle feinfühlig einstellbar und fixierbar ist.

Vorteilhafte Ausgestaltungsformen der Erfindung sind in abhängigen Patentansprüchen gekennzeichnet.

Mit der erfindungsgemäßen Klemmvorrichtung können Meßwertaufnehmer, insbesondere für polarographische Messungen an der Innenseite der Wange oder an der Innenseite der Unterlippe mit genau definiertem Anpreßdruck festgeklemmt werden.

Die Erfindung und vorteilhafte Einzelheiten werden nachfolgend unter Bezug auf die Zeichnung in mehreren Ausführungsbeispielen erläutert. Es zeigt

Fig. 1 in schematischer Darstellung eine erste Ausführungsform einer Klemmvorrichtung mit Merkmalen nach der Erfindung,

Fig. 2 ein weiteres Ausführungsbeispiel der Erfindung, das sich besonders zur Anwendung bei räumlich sehr begrenzten Verhältnissen eignet und sich durch eine geringe Biegemomentbelastung der Applikationsstelle auszeichnet,

Fig. 3 in schematischer Darstellung eine mögliche Konstruktionseinzelheit der erfindungsgemäßen Klemmvorrichtung,

Fig. 4 eine abgewandelte Klemmvorrichtung mit erfindungsgemäßen Merkmalen und

Fig. 5 eine weitere Ausführungsform einer erfindungsgemäßen Klemmvorrichtung, die sich durch eine besonders gute Einstellbarkeit des Anpreßdrucks für den Meßwertaufnehmer auszeichnet.

Einander entsprechende Teile sind in den einzelnen Figuren mit den gleichen Bezugshinweisen gekennzeichnet.

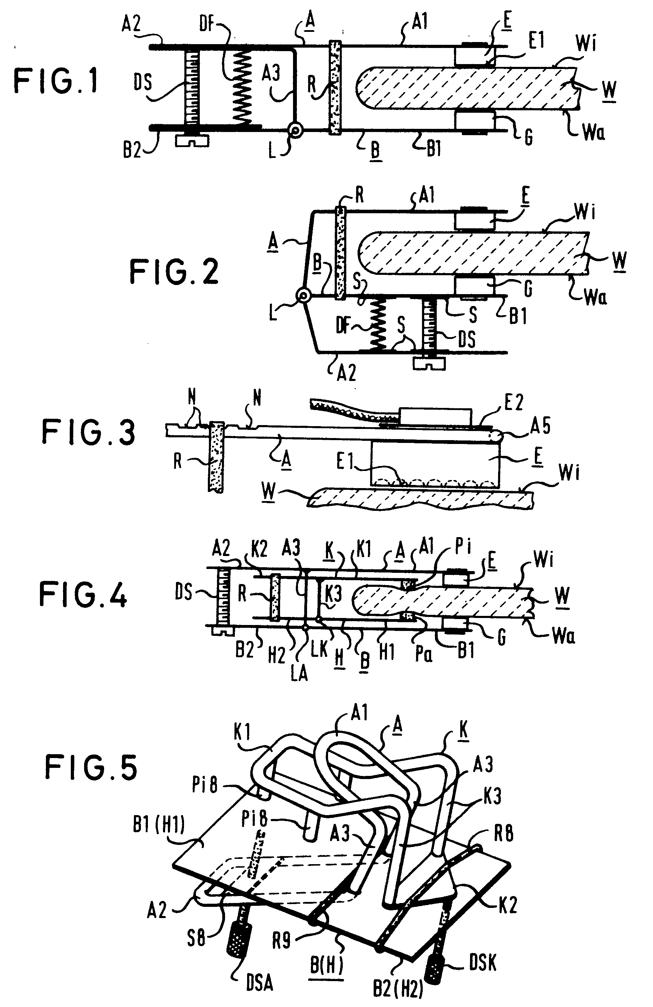

Bei der ersten Ausführungsform der Klemmvorrichtung nach Fig. 1 sind zwei auf Abstand voneinander gehaltene Klemmarme mit A und B bezeichnet und gegenseitig verschwenkbar im Drehlager L aneinander angelenkt. An dem an der Innenseite Wi der Wange oder der Unterlippe W anzuordnenden Klemmenende A1 wird der Meßwertaufnehmer E so befestigt, daß seine Meßfläche E1 der Innenseite der Wange oder Lippe zugekehrt ist. Es ist zweckmäßig, am anderen Ende B1 des an der Außenseite Wa anzulegenden Klemmarms B ein Gegenlager G für den Aufnehmer E zu befestigen, das in Flächenausdehnung und Querschnitt etwa dem Meßwertaufnehmer E gleicht. Anstatt ein zusätzliches Teil daran zu befestigen, kann das Klemmenende B1 zur Bildung des Gegenlagers G auch einfach scheibenförmig ausgebildet sein.

Der Klemmarm A, dessen Klemmende A1 bei der Applikation des Meßwertaufnehmers E an der Mundinnenseite zu liegen kommt, ist wippenartig derart ausgebildet, daß er sich nicht mit dem außenliegenden Klemmarm B kreuzt, sondern mittels eines Stützarms A3, der im wesentlichen senkrecht zum Klemmarm A verläuft, am Klemmarm B im Lager L angelenkt ist. Für die Aufbringung eines Teils des Klemmdrucks kann beispielsweise ein Gummiring R über die außenliegenden Klemmarmenden geschoben sein oder es kann zwischen den außenliegenden Bedienungsenden A2 und B2 eine Druckfeder DF angebracht sein. Zur präzisen Einstellung des Klemmdrucks ist jedoch eine Druckschraube DS drehbar in das Bedienungsende B2 eingeschraubt, die gegen das gegenüberliegende Bedienungsende A2 des anderen Klemmarms A anstellbar ist, so daß der Klemmdruck einfach durch Verdrehen der Druckschraube DS in gewissen Grenzen der Wangen- oder Lippendicke entsprechend eingestellt und gehalten werden kann, so daß der über die genannten Bedienungsenden gestreifte Gummiring R nur eine allgemeine Haltefunktion übernimmt. Die Kraft für Halterung und der Anpreßdruck sind feinfühlig einstellbar, und zwar einerseits durch Wahl der Lage des Gummirings R in verschiedenen Abständen vom Drehlager mit Präzisionseinstellung durch die drehbar gelagerte Druckschraube DS, die beim Einschrauben mit ihrem feinen Anschlagende versucht, den Bedienungsarm A2 wegzudrücken.

Bei der Ausführungsform nach Fig. 2 ist der Klemmarm A im Prinzip ebenfalls wippenartig ausgebildet und ist über das Drehlager L hinaus verlängert und in Richtung des Lagers L zum einarmig abstehenden Klemmarm B umgebogen. Der umgebogene und verlängerte, den Bedienungsarm A2 bildende Teil ist bei Wahrung eines zur Bedienung ausreichenden Abstands vom Klemmarm B in Richtung zu diesem so umgebogen, daß die Bedienungsenden einerseits vom über das Drehlager L hinausstehenden Klemmarmteil A2 und andererseits durch den Klemmarm B gebildet sind. Zwischen diesen Bedienungsenden kann dann wieder die Druckfeder DF sowie die Druckschraube DS angeordnet sein, um einen geeigneten Klemmdruck aufzubringen und den Abstand zwischen dem Meßwertaufnehmer E und dem Gegenlager G der Wangendicke entsprechend fein reguliert einstellen zu können. Diese Ausführungsform hat den Vorteil, daß der eine überstehende Klemmarmteil B2 entfällt, so daß die gesamte Klemmvorrichtung ein geringeres Gewicht als bei der Ausführungsform nach Fig. 1 hat und überdies kein nennenswertes Moment auf die Applikationsstelle durch die außen vorstehenden Teile der Klemmarme gegeben ist. Die Handlichkeit der Klemmvorrichtung ist ähnlich gut wie bei der Ausführungsform nach Fig. 1.

Die Fig. 3 zeigt eine Konstruktionseinzelheit bei einer möglichen Ausgestaltungsform der Erfindung. Die Klemmarme der Klemmvorrichtung sollen ein möglichst geringes Gewicht aufweisen; sie sollen trotzdem formbeständig und nicht leicht verbiegbar sein. Zweckmäßigerweise bestehen sie daher aus einem Bügel aus Kunststoff, aus Metall, insbesondere aus Edelstahl oder aus oberflächenvergütetem Metall in Form eines länglich ovalen Rings. Für die Befestigung des Meßwertaufnehmers E kann dieser auf seiner der Meßfläche E1 abgewandten Seite E2 mit einer Umlaufrille versehen sein, die zur Fassung im Bügelteil A5 dient. An den Stellen, an denen die Druckschraube DS, eine Druckfeder DF oder dergleichen angreifen, werden zweckmäßig zwischen den gegenüberliegenden Zonen des Bügels Plättchen oder Scheiben eingespannt, die die Lager bzw. Gegenlager für die Feder DF bzw. Druckschraube DS bilden. In verschiedenen Abständen vom Drehlager L können Nuten oder Rillen N angebracht sein, damit für die Halterung des zusätzlich benutzten Gummirings R unverrückbar ein geeigneter Abstand vom Drehlager L festgelegt wird. Die sonstige Formgebung der Halterungsvorrichtung und der Klemmarme sollte den Verhältnissen an der Applikationsstelle angepaßt sein, z. B. mit dem Ziel, daß die Arme möglichst wenig vom Gesicht abstehen, was bei der Ausführungsform nach Fig. 2 verwirklicht ist. Für den Meßwertaufnehmer E kann es zweckmäßig sein, die Meßfläche E1, die in Berührung mit der Mundschleimhaut kommt, nicht eben auszubilden, sondern mit umlaufenden Rillen und Wülsten zu versehen, so daß eine bessere Abdichtung des bedeckten Teils der Mundschleimhaut gegenüber dem Mundspeichel erreicht wird. Dies wird bei der Ausführungsform nach Fig. 3 durch eine wellige Gestaltung der Meßfläche E1 angedeutet.

Die Länge der einzelnen Klemmarme A, B, der Klemmenden A1, B1 und der Bedienungsenden A2, B2 wird so bemessen, daß eine gute Anbringung ermöglicht und die gewünschte Funktion mit ausreichendem Andruck des Meßwertaufnehmers E erreicht wird, ohne daß die Bedienungsenden hinderlich und störend sind. Die Einstellbarkeit des Klemmdrucks, die mit der erfindungsgemäßen Klemmvorrichtung durch die Druckschraube DS möglich ist, ist physiologisch von großer Bedeutung, denn ein zu großer Klemmdruck kann zu einer Unterbindung der Durchblutung führen, ein zu kleiner Klemmdruck dagegen kann Anlaß zu erheblichen Fehlern bei der Messung sein. Die reine Haltefunktion übernimmt zweckmäßig der über die Klemmenden zu streifende Gummiring R in passendem Abstand vom Drehlager L.

Eine Weiterentwicklung des Erfindungsgedankens besteht darin, daß — wie die schematische Darstellung der Fig. 4 zeigt — die Funktion der Anklammerung der Klemmvorrichtung an der Wange oder Lippe W von der Funktion der Halterung des Aufnehmers E getrennt wird. Die Fig. 4 zeigt, wie die vorhergehend beschriebenen Ausführungsformen, einen Querschnitt durch die Wange W mit den angelegten Klemmarmen A, B. Dazu kommen jedoch noch weitere Klemmarme K und H in wippenartiger Ausführung etwa gemäß Fig. 1. Zur Halterung der gesamten Klemmvorrichtung an der Wange oder die Lippe W dienen besondere Klammerarme K, H mit den wangenseitigen Armteilen K1, H1 und den bedienungsseitigen Armteilen K2, H2. Der Arm K stützt sich mit dem Armteil K3 auf den anderen Arm H am Lager LK ab, das aus einem einfachen Schlitz in der entsprechenden Stützplatte S bestehen kann. Die gegenseitige Anlenkung der beiden Klammerarme kann aber auch nur aus einer elastischen oder federnden Verbindung zwischen ihnen bestehen.

Während der Anpreßdruck für die Klammerarme K1, H1 so stark sein kann, daß die Wange — wie in Fig. 4 gezeigt — durch die Armpolster Pi, Pa zusammengedrückt wird, wäre dies für die Messung nicht sinnvoll, weil dadurch die Durchblutung der Meßzone der Wange behindert würde. Deshalb übernimmt zweckmäßig allein das Klammerarmpaar K, H die Halterung der Vorrichtung, während die Klemmarme A, B eine Vorrichtung aufweisen, die einen wesentlich geringeren Anpreßdruck für den Meßwertaufnehmer E und für das Gegenlager G erzeugen. Diese Vorrichtung besteht erfindungsgemäß wiederum in einer im Armteil B2 drehbar gelagerten und axial gegenüber dem Armteil A2 durch Verdrehen verdrehbaren Druckschraube DS. Der Anpreßdruck für den Aufnehmer E kann also sehr fein einreguliert werden. Für die Handhabung ist es zweckmäßig, daß dabei die Klammerarme und die Klemmarme nebeneinander angeordnet werden, vorteilhafterweise auch symmetrisch, so daß Klammerpaare, und zwar auf jeder Seite des Klemmarmpaares je ein Paar, vorhanden sind.

Um zu verhindern, daß die Klemmarme A1, B1 sich im nicht applizierten Zustand ganz schließen, was zu einer Beschädigung des Meßaufnehmers führen könnte, kann an den Klemmarmen eine Sperre vorhanden sein, etwa in Form eines einfachen Anschlags oder in Form eines Blattfederelements an einem oder beiden der Klemmarme.

Die Fig. 5 zeigt eine weitere Ausführungsform einer Halterungsvorrichtung für Meßwertaufnehmer nach dem in Fig. 4 schematisch dargestellten Konstruktionsprinzip. Dabei wird der Klemmarm K wie auch der Klemmarm A, der, ähnlich wie in Fig. 3 dargestellt, zur Halterung des hier nicht gezeigten Meßwertaufnehmers dient, aus je einem länglich ovalen Drahtbügel gebildet, während die Funktion der Gegenarme B und H von einer gemeinsamen, an der Wangen- oder Lippenaußenseite anzuordnenden Grundplatte B(H) übernommen wird. Der Klammerarm K und der Klemmarm A wirken wippenartig, indem jeweils ihr Endteil K3 bzw. A3 in Richtung zur Grundplatte B(H) umgebogen und diese Teile als Wippenstützen auf der Grundplatte B(H) drehbar angelegt sind. Dabei sind die Bügelteile des Klemmarms A durch die Grundplatte B(H) fortgesetzt und bilden den Armteil A2 auf der anderen Seite der Grundplatte, während die beiden Bügelteile K3 des Klemmarms K auf der Oberseite der Grundplatte B(H) durch den plättchenartigen Klammerteil K2 verbunden sind. Dieser Klammerteil K2 bildet das Bedienungsende zum Verstellen des Klammerandrucks. Dazu ist erfindungsgemäß eine Druckschraube DSK im Teil B2(H2) der Grundplatte drehbar gelagert, deren aus der Grundplatte herausragendes Endstück gegen die Unterseite des Bügelteils drückt und somit je nach der Einschraublänge den gesamten Klammerarm K an die Mundinnenseite andrückt. Bei dem Klemmarm A ist eine zweite Druckschraube DSA nicht in der Grundplatte sondern an dem an ihrer Außenseite fortgesetzten Bedienungsteil A2 des Klemmarms A drehbar gelagert, wozu die beiden Bügelhälften durch ein Plättchen S8 überbrückt sind. Das Ende des Schraubschafts der Druckschraube DSA kann somit gegen die Außenseite der Grundplatte angestellt werden, wodurch sich der Anpreßdruck für die im Armteil A1 zu befestigende Elektrode an die Mundschleimhaut andrücken läßt. Das Klammerende K1 des Klemmarms K trägt drei Abstützelemente Pi8. Die Voreinstellung des Anpreßdrucks kann durch einen Gummiring R9 unterstützt sein, der über die Grundplatte und das Bedienungsende A2 des Klemmarmbügels A zu schieben ist.

## Patentansprüche

1. Klemmvorrichtung zur Halterung von biologisch-physiologischen Meßwertaufnehmern und/oder Elektroden im Munde, insbesondere im Bereich der Unterlippe oder einer Wange mit
— zwei gegeneinander verschwenkbaren miteinander verbundener Klemmarme (A, B), von denen wenigstens der eine in Form einer Wippe gestaltet und an seinem einen Klemmarmende (A1) zur Befestigung des an einer Mundinnenseite (Wi) zu applizierenden Meßwertaufnehmers (E) eingerichtet und das zur Applikationsstelle korrespondierende Klemmarmende (B1) des anderen Klemmarms (B) als Gegenlager (G) an der Mundaußenseite anzulegen ist sowie mit
— Elementen (R, ZF, DF, DS) zur Aufbringung eines vorgebbaren Klemmdrucks zwischen den beiden Klemmarmenden (A1, B1) an der Applikationsstelle,
dadurch gekennzeichnet, daß
— in das außerhalb des Mundes liegende Bedienende (B2 oder A2) eines der Klemmarme (B oder A) eine drehbare und damit axial verstellbare Druckschraube (DS; DSA; DSK) eingesetzt ist, die gegen das gegenüberliegende Ende des anderen Klemmarms oder gegen eine beiden Klemmarmen gemeinsame Halteplatte (B[H]) so anstellbar ist, daß ein geeigneter Klemmdruck des Meßwertaufnehmers (E) gegen die Applikationsstelle feinfühlig einstell- und fixierbar ist.

2. Klemmvorrichtung nach Anspruch 1, dadurch gekennzeichnet, daß die beiden Klemmarme (A, B) durch ein gemeinsames Gelenk (L) miteinander verbunden sind.

3. Klemmvorrichtung nach Anspruch 1, dadurch gekennzeichnet, daß das Schwenkgelenk des einen, in Form einer Wippe gestalteten Klemmarms (A) in der Ebene der Halteplatte (B[H]) liegt, und daß am von der Applikationsstelle auf der Außenseite der Halteplatte angeordneten Ende dieses Klemmarms die Druckschraube (DSA) angebracht und gegen die Halteplatte anstellbar ist.

4. Klemmvorrichtung nach Anspruch 2, dadurch gekennzeichnet, daß nur der an der Mundinnenseite (Wi) anzuordnende und den Meßwertaufnehmer (E) tragende Klemmarm (A) über das gemeinsame Gelenk (L) hinaus verlängert und in einem Abstand in Erstreckungsrichtung des außenliegenden Klemmarms (B) umgebogen ist, so daß die Bedienungsenden vom außenliegenden Klemmarmende (B1) einerseits und vom umgebogenen außenliegenden und über das Gelenk (L) hinaus verlängerten Klemmarmende (A2) des den Meßaufnehmer (E) tragenden Klemmarms andererseits gebildet sind, und daß die Druckschraube (DS) im umgebogenen verlängerten Klemmarmende (A2) gelagert und gegen das gegenüberstehende, auf der Mundaußenseite angeordnete andere Klemmarmende (B1) anstellbar ist.

5. Klemmvorrichtung nach Anspruch 1, dadurch gekennzeichnet, daß die Klemmarme (A, B) aus Bügeln bestehen, die zu einem länglich ovalen Ring oder Teilring verformt sind, und daß die klemmseitigen Enden (A5) des Bügels des an der Mundinnenseite (Wi) zu applizierenden Klemmarms (A) als Fassung für den Meßwertaufnehmer (E) dienen, der an seiner der Meßfläche (E1) entgegengesetzten Seite eine umlaufende angepaßte Rille aufweist.

6. Klemmvorrichtung nach Anspruch 5, dadurch gekennzeichnet, daß die Bügel der Klemmarme aus Kunststoff, Metall, insbesondere Edelstahl oder oberflächenvergütetem Metall gefertigt sind.

7. Klemmvorrichtung nach Anspruch 1, dadurch gekennzeichnet, daß an dem an der Mundaußenseite (Wa) zu applizierenden Klemmarmende (B1) eine in Ausdehnung und Form der Anlagefläche des Meßwertaufnehmers (E) ähnliche Scheibe (G) als Gegenlager angebracht ist.

8. Klemmvorrichtung nach Anspruch 1, dadurch gekennzeichnet, daß das Klemmende (B1) des einen Klemmarms (B), das nicht zur Befestigung des Meßwertaufnehmers (E) dient, zur Bildung eines Gegenlagers für den Meßaufnehmer (E) scheibenförmig ausgebildet ist.

9. Klemmvorrichtung nach Anspruch 1, dadurch gekennzeichnet, daß zur Aufbringung eines Klemmdrucks auf die Klemmenden (A1, B1) an der Applikationsstelle eine Druckfeder (DF) zwischen den Bedienungsenden der Klemmarme eingespannt ist.

## Claims

1. A clamping device for holding biological-physiological measurement receivers and/or elektrodes in the mouth, particularly in the region of the lower lip or a cheek, comprising
— a pair of connected clamping arms (A, B) pivotally movable with respect to each other, at least one of said arms being designed in form of a whip and being furnished at the one end of the clamping arm (A1) to fix the measurement receiver (E) to be applied to one inner side of the mouth (Wi), and the clamping arm end (B1) of the other clamping arm (B) corresponding to the application point is to be applied to the outside of the mouth as outer support (G), as well as
— elements (R, ZF, DF, DS) for applying a predeterminable clamping pressure between the pair of clamping arm ends (A1, B1) at the application point,
characterized in that
— a pivotally and thereby axially adjustable setscrew (DS; DSA; DSK) is positioned in the operating end (B2 or A2) outside the mouth of one of said clamping arms (B or A), which setscrew is adjusted against the opposite end of the other clamping arm or against a support plate (B[H]) common to both clamping arms such that a suitable clamping pressure of said measurement receiver (E) against said application point may be adjusted and fixed.

2. A clamping device according to claim 1, characterized in that said pair of clamping arms (A, B) is connected by a common joint (L).

3. A clamping device according to claim 1, characterized in that the pivotable joint of the one clamping arm (A) designed in the form of a whip is arranged in the coplanar of said support plate (B[H]), and that said setscrew (DSA) is located at the end of said clamping arm positioned outside said support plate at the application point and is adjustable against said support plate.

4. A clamping device according to claim 2, characterized in that only said clamping arm (A) to be arranged at the inside of the mouth (Wi) and carrying said measurement receiver (E) is extended over said common joint (L) and bent in a distance in the direction of extension of the clamping arm (B) positioned at the outside so as to form the operating ends of the external clamping arm end (B1), on the one hand, and of the bent exterior clamping arm end (A2) extending over said joint (L) of the clamping arm carrying said measurement receiver (E), on the other hand, and that said setscrew (DS) is mounted in the bent extended clamping arm end (A2) and is adjustable against the opposite other clamping arm end (B1) positioned at the outside of the mouth.

5. A clamping device according to claim 1, characterized in that said clamping arms (A, B) consist of clips shaped to an oval ring or graduated ring, and that the ends (A5) at the clamping sides of said clip of said clamping arm (A) to be applied to the inside of the mouth (Wi) serve as mounting for said measurement receiver (E) having a circumferential adapted groove on the side opposite the measurement area (E1).

6. A clamping device according to claim 5, characterized in that the clips of said clamping arms are formed of plastics, metal, particularly noble metal or a surface heat-treated metal.

7. A clamping device according to claim 1, characterized in that disc (G) similar in its extent and shape to the application area of said measurement receiver (E) is applied as outer support to the clamping arm end (B1) to be applied at the outside of the mouth (Wa).

8. A clamping device according to claim 1, characterized in that said clamping end (B1) of said clamping arm (B) not serving to fix said measurement receiver (E) is designed disc-shaped so as to form an outer support for said measurement receiver (E).

9. A clamping device according to claim 1, characterized in that a setscrew (DF) is clamped between the operating ends of said clamping arms to thereby apply a clamping pressure to the clamping ends (A1, B1) at the application point.

## Revendications

1. Dispositif de serrage pour la fixation de transducteurs biologiques-physiologiques et/ou d'électrodes dans la bouche, en particulier dans la région de la lèvre inférieure ou de la joue avec

— deux bras de serrage (A, B), qui sont attachés l'un avec l'autre et qui peuvent pivoter l'un contre l'autre et dont un au moins est réalisé en forme de bascule et est aménagé à l'une de ses extrémités (A1) pour le fixage due transducteur (E) prévu d'être appliqué à la face interne (Wi) de la bouche, l'extrémité (B1) du bras de serrage correspondant à l'endroit d'application de l'autre bras de serrage (B) ètant prévue pour être mise contre la face extérieure de la bouche comme butée (G), et avec

— des èléments (R, ZF, DF, DS) pour la production d'une pression de serrage, fixée d'avance, entre les deux extrémités (A1, B1) de bras de serrage à l'endroit de l'application,

caractérisé en ce que

— une vis de pression (DS, DSA, DSK) rotative et par cela axialement réglable est placée dans l'extrémité de commande (B2 ou A2) située à l'extérieur de la bouche et peut être mise en place contre l'extrémité opposée de l'autre bras de serrage ou contre une platine de support (B[H]) commune aux deux bras de serrage de manière qu'une pression de serrage appropriée du transducteur (E) peut être réglée et bloquée d'une manière sensible.

2. Dispositif de serrage selon la revendication 1, caractérisé en ce que les deux bras de serrage (A, B) sont attachés l'un à l'autre par un joint (L) commun.

3. Dispositif de serrage selon la revendication 1, caractérisé en ce que le joint pivotant de l'un des bras de serrage (A), qui est réalisé en forme de bascule, est situé dans le plan de la platine de support (B[H]) et que la vis de pression (DSA) est installée à l'extrémité de ce bras de serrage du côté extérieur de la platine de support vue de l'endroit de l'application et peut être mise in place contre la platine de support.

4. Dispositif de serrage selon la revendication 2, caractérisé en ce que seulement le bras de serrage (A) prévu pour être placé du côté intérieur de la bouche (Wi) et portant le transducteur (E) est prolongé au delà du joint (L) commun et est replié, à une certaine distance dans la direction d'extension du bras de serrage (B) situé à l'extérieur, de façon que les extrémités de commande sont formées d'une part de l'extrémité du bras de serrage (B1) située à l'extérieur, et d'autre part de l'extrémité du bras de serrage (A2) replié et situé à l'extérieur et prolongé au delà du joint (L) et portant le transducteur (E), et que la vis de pression (DS) est logée dans l'extrémité du bras de serrage (A2) repliée et prolongée et peut être mise en place contre l'autre extrémité de bras de serrage (B1) se trouvant en face du côté extérieur de la bouche.

5. Dispositif de serrage selon la revendication 1, caractérisé en ce que les bras de serrage (A, B) consistent d'étriers qui sont déformés en anneaux ovales ou anneaux partiels et en ce que les extrémités (A5) du côté de serrage de l'êtrier du bras de serrage (A) prévu pour l'application sur la face interne de la bouche (Wi) servent comme monture pur le transducteur (E), qui présente du côté opposé à sa surface de mesure une rainure adaptée le contournant.

6. Dispositif de serrage selon la revendication 5, caractérisé en ce que les étriers des bras de serrage sont manufacturés en matière synthétique, en métal, en particulier acier spécial ou en métal dont la surface a été traitée.

7. Dispositif de serrage selon la revendication 1, caractérisé en ce que sur l'extrémité du bras de serrage (B1) prévue d'être appliquée sur la face externe de la bouche (Wa) est disposé un plateau (G) ayant une étendue et une forme semblable à celles du transducteur (E) et servant somme butée.

8. Dispositif de serrage selon la revendication 1, caractérisé en ce que l'extrémité (B1) du bras de serrage (B), qui ne sert pas pour la fixation du transducteur (E), est façonnée en forme de disque pour la formation d'une butée pour le transducteur (E).

9. Dispositif de serrage selon la revendication 1, caractérisé en ce qu'un ressort à pression (DF) est serré à l'endroit de l'application entre les extrémités de commande des bras de serrage pour la production d'une pression de serrage sur les extrémités de bras de serrage (A1, B1).

FIG.1

FIG.2

FIG.3

FIG.4

FIG.5